# EUROPEAN PATENT APPLICATION

(11) **EP 4 067 396 A1**
(43) Date of publication of application: **05.10.2022**
(21) Application number: 20891558.7
(22) Date of filing: 25.11.2020
(51) Int. Cl.: C08F 220/36, G01N 29/02

(54) **COPOLYMER, DETERMINATION DEVICE, AND CARRIER FOR DETERMINATION**

(30) Priority: 28.11.2019 JP 2019215690
(71) Applicant: Kyocera Corporation, Kyoto-shi Kyoto 612-8501 (JP)
(72) Inventor: KURIOKA, Hideharu, Kyoto-shi, Kyoto 612-8501 (JP)
(74) Representative: TBK
(86) International application number: PCT/JP2020/043728
(87) International publication number: WO 2021/106899

(57) **Abstract**

Solution

A copolymer according to the present disclosure is a copolymer of a compound represented by Formula (1) and a compound represented by Formula (2). R¹ and R⁴ are each independently a hydrogen atom or a methyl group. R² and R³ are each independently a hydrogen atom or an alkyl group having from 1 to 4 carbon atom(s). R⁵ and R⁶ are each independently an alkyl group having from 1 to 4 carbon atom(s). x, y, and z are each independently an integer from 1 to 4.

## Description

### Technical Field

The present disclosure relates to a copolymer that forms a polymer film used in a measuring device, a measuring device provided with a polymer film, and a carrier for measurement.

### Background Art

Patent Document 1 discloses a surface acoustic wave sensor as an example of a measuring device for measuring the concentration of a target measurement substance (e.g., a biomolecule). Such a measuring device includes a detection unit, in which a substance (e.g., an antibody) that interacts with a target measurement substance contained in a sample is fixed. A polymer film is often formed on the detection unit for the purpose of reducing non-specific adsorption of substances other than the target measurement substance contained in the sample.

### Citation List

### Patent Literature

Patent Document 1: JP 2008-286606 A

### Summary

### Technical Problem

It is desirable that the polymer film mentioned above has a non-specific adsorption property that differs depending on the application of the polymer film, such as the type of the target measurement substance, the measurement precision, and the measurement method.

### Solution to Problem

A copolymer according to an embodiment is a copolymer of a compound represented by Formula (1) and a compound represented by Formula (2): 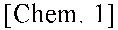 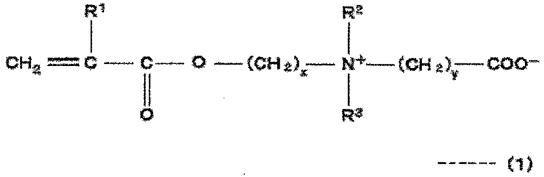 where in Formula (1),
R¹ is a hydrogen atom or a methyl group,
R² and R³ are each independently a hydrogen atom or an alkyl group having from 1 to 4 carbon atom(s), and
x and y are each independently an integer from 1 to 4 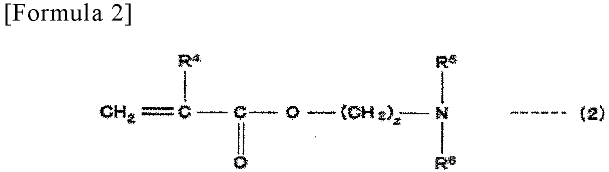 where in Formula (2),
R⁴ is a hydrogen atom or a methyl group,
R⁵ and R⁶ are each independently an alkyl group having from 1 to 4 carbon atom(s), and
z is an integer from 1 to 4.

### Advantageous Effects of Invention

According to the present disclosure, a polymer film having a different non-specific adsorption property can be produced.

### Brief Description of Drawings

FIG. 1 is a schematic view illustrating an example of a measuring device according to 10.
FIG. 2 is a plan view illustrating a sensor provided in the measuring device.
FIG. 3 is a cross-sectional view along the line A-A of the sensor of the measuring device illustrated in FIG. 1.
FIG. 4 is a conceptual diagram illustrating a polymer provided in a detection unit 23 illustrated in FIG. 1.
FIG. 5 is a plan view illustrating an example of a carrier for measurement according to the present disclosure.
FIG. 6 is a conceptual diagram illustrating a polymer film formed in a non-detection region of the carrier for measurement.
FIG. 7 is a graph illustrating a relationship between a proportion of DMAEMA units in the polymer prepared in Example 1 and an amount of polymer film formed.
FIG. 8 is a graph illustrating a relationship between a proportion of DMAEMA units in the polymer prepared in Example 1 and an amount of non-specific adsorption of serum.
FIG. 9 is a graph illustrating a relationship between a proportion of DMAEMA units contained in the polymer and a hydrolysis time.
FIG. 10 is a diagram illustrating an analysis result of polymer by NMR spectroscopy.
FIG. 11 is a graph illustrating an examination result of the polymerization conditions in Evaluation Example 4.
FIG. 12 is a graph illustrating a relationship between a molecular weight of polymer and an amount of non-specific adsorption of serum or urine.

### Description of Embodiments

### Embodiment 1

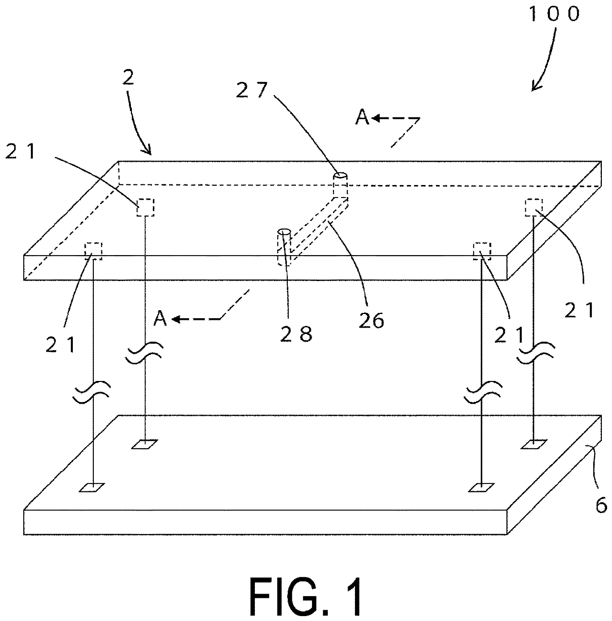

Hereinafter, a sensor provided in a measuring device according to an embodiment will be described using drawings when appropriate. FIG. 1 illustrates a schematic view of a sensor 2 of a measuring device 100 according to the present embodiment.

The measuring device 100 can target and detect a specific substance (first substance) in a measurement target (sample). Examples of the measurement target include biological samples, such as blood samples including serum and plasma, and body fluids including sweat, urine, tears, and saliva. The first substance 5 is, for example, a substance in a living organism. Examples of the substance in a living organism include proteins, DNAs, and substrates for enzymatic reaction. The measuring device 100 is provided with the sensor 2 that can detect the first substance and a control device 6 that can control the measuring device 100.

The sensor 2 may be a sensor that utilizes, for example, acoustic waves, Quartz Crystal Microbalance (QCM), Surface Plasmon Resonance (SPR), or Field Effect Transistors (FETs). That is, the sensor 2 may be any as long as it is one that is able to convert an electrical signal to and from an acoustic wave, QCM, SPR, or FET. The sensor 2 according to an embodiment is a sensor that utilizes acoustic waves. That is, by using the sensor 2, the measuring device 100 according to an embodiment can detect the change in the acoustic wave based on the presence of the first substance as a change in the electrical signal. The sensor 2 may be produced by a known method.

In the measuring device 100 according to the present embodiment, the sensor 2 includes an external terminal 21. The sensor 2 can be electrically connected, through the external terminal 21, to the control device 6 that controls the measuring device 100. That is, the sensor 2 and the control device 6 can input and output electrical signals from and to each other through the external terminal 21. As such, the control device 6 can, for example, detect the first substance based on an electrical signal input from the sensor 2. The control device 6 may, for example, calculate the concentration of the first substance contained in the sample. Alternatively, the control device 6 may, for example, identify the first substance. The control device 6 and the external terminal 21 may be produced using a known technique. In addition, the configuration of electrical connection between the sensor 2 and the control device 6 is not limited to the external terminal 21. For example, as long as the sensor 2 and the control device 6 can be electrically connected, the sensor 2 and the control device 6 do not need to be physically connected through a terminal or the like. For example, the sensor 2 and the control device 6 may be electrically connected by electromagnetic induction.

The sensor 2 may be a disposable cartridge. This makes it unnecessary to clean the sensor 2 after measurement, and the effect on the measurement result due to insufficient cleaning can be eliminated.

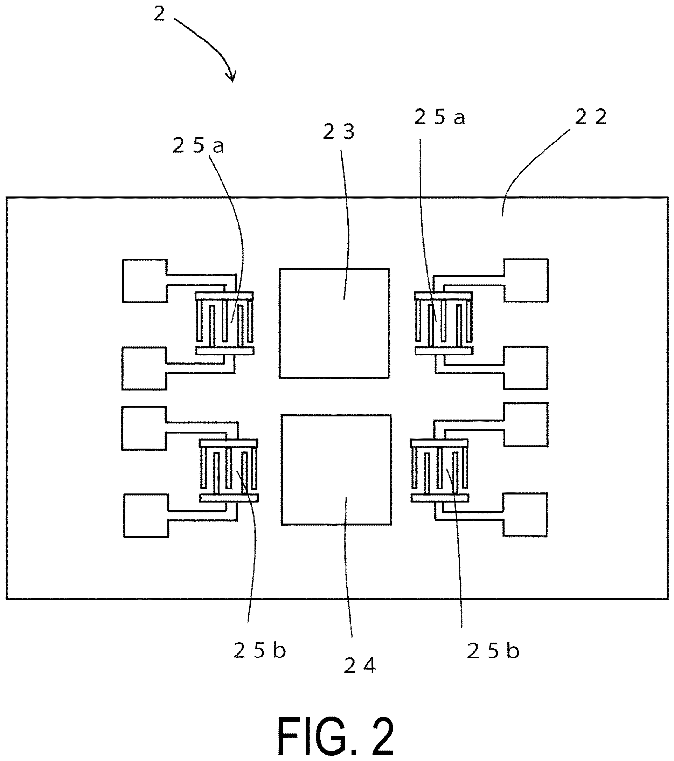

FIG. 2 illustrates a plan view of the sensor 2. The sensor 2 is provided with a substrate 22, a detection unit 23 disposed on the substrate 22, and a pair of first inter digital transducer (IDT) electrodes 25a arranged on the substrate 22 sandwiching the detection unit 23. The substrate 22 can support the first IDT electrodes 25a and the like.

### Substrate

The substrate 22 is a piezoelectric substrate. For example, the substrate 22 may be a substrate containing a metal such as gold, silver, copper, platinum, and aluminum, or a piezoelectric single crystal such as lithium tantalate and crystal. In the present embodiment, the substrate 22 is a piezoelectric substrate containing crystal.

### Detection unit

A substance (second substance) that reacts with the first substance 5 is fixed to the detection unit 23. Thus, the first substance contained in the sample can react with the second substance fixed to the detection unit 23 in the detection unit 23. By the reaction between the first substance and the second substance, the detection unit 23 can change the propagation characteristics of acoustic waves of the substrate 22. Specifically, the detection unit 23 can, for example, change the weight applied to the substrate 22 or the viscosity of a liquid in contact with a surface of the substrate 22 by the reaction between the first substance and the second substance. Accordingly, the phase, amplitude, period, or the like of the acoustic waves propagating in the substrate 22 changes. Thus, the sensor 2 can detect a change in the surface acoustic waves corresponds to the concentration of the first substance. The details of the detection unit 23 will be described later.

The pair of first IDT electrodes 25a can generate acoustic waves between the pair of first IDT electrodes 25a. Of the acoustic waves generated, the acoustic waves propagating at the surface of the substrate 22 may be referred to as surface automatic waves (SAWs). In the measuring device 100 according to an embodiment, an electrical signal is input from one of the pair of first IDT electrodes 25a. The input electrical signal is converted to an acoustic wave propagating towards the detection unit 23, which is then emitted from the one IDT electrode 25a. The emitted acoustic wave passes through the detection unit 23. The other IDT electrode 25a can receive the acoustic wave that has passed through the detection unit 23. The received acoustic wave is converted to an electrical signal. The pair of first IDT electrodes 25a may be formed of a metal material such as gold, chromium, or titanium. In addition, the pair of first IDT electrodes 25a may be single-layer electrodes including a single material, or multi-layer electrodes including a plurality of materials.

The sensor 2 may have two or more groups of the detection unit 23 and the pair of IDT electrodes 25a. In this case, for example, each group in the measuring device 100 may detect a different type of target substance. Alternatively, the measuring device 100 may, for example, detect the same type of target substance using a plurality of the groups and compare the detection results from each of the groups.

### Reference unit

The sensor 2 may have a reference unit 24 disposed on the substrate 22. Additionally, the sensor 2 may include a pair of second IDT electrodes 25b disposed on the substrate 22 sandwiching the reference unit 24. In the measuring device 100 according to an embodiment, an electrical signal is input from one of the pair of first IDT electrodes 25b. The input electrical signal is converted to an acoustic wave propagating towards the reference unit 24, which is then emitted from the one IDT electrode 25b. The emitted acoustic wave passes through the reference unit 24. The other IDT electrode 25b can receive the acoustic wave that has passed through the reference unit 24. The received acoustic wave is converted to an electrical signal.

The reference unit 24 can function as a control of the detection unit 23. Specifically, unlike in the detection unit 23, the second substance is not fixed to the reference unit 24. That is, the reaction between the first substance and the second substance does not occur in the reference unit 24. Thus, the measuring device 100 can use a reference signal, which is an electrical signal based on an acoustic wave that has passed through the reference unit 24, as a reference to detect the first substance based on a detection signal, which is an electrical signal based on an acoustic wave that has passed through the detection unit 23. Specifically, if a sample contains the first substance, the first material reacts with the second substance, generating a difference between the detection signal and the reference signal. Thus, for example, the measuring device 100 can detect the first substance by calculating the difference between the detection signal and the reference signal. In addition, for example, the measuring device 100 can detect a change in the reaction between the first substance and the second substance by detecting a change in the difference between the detection signal and the reference signal.

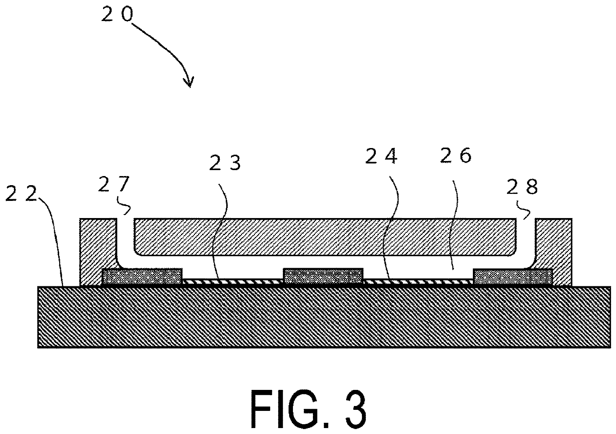

FIG. 3 illustrates a cross-sectional view of the sensor 2 along line A-A at a cross-sectional plane in FIG. 1. The sensor 2 further includes a flow path member 26. The flow path member 26 can function as a passage of sample. The flow path member 26 includes a supply port 27 for supplying a sample and a discharge port 28 for discharging the sample, the supply port 27 and the discharge port 28 both opening on the upper surface of the flow path member 26. In the sensor 2, when a sample supplied from the supply port 27 reaches the detection unit 23, detection of the first substance occurs in the detection unit 23; then, the sample is discharged from the discharge port 28.

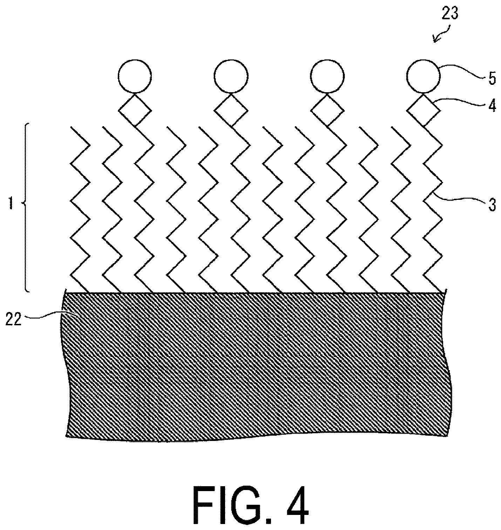

FIG. 4 illustrates a conceptual diagram of a polymer film 1 included in the detection unit 23. In the detection unit 23, the polymer film 1 containing a polymer 3 is fixed to the substrate 22. Furthermore, the substance (second substance 4) that reacts with the first substance 5 is fixed to the polymer film 1.

The polymer film 1 is a film adjusted to have a high specific adsorption property. That is, the polymer film 1 is a film adjusted to have a reduced non-specific adsorption. The non-specific adsorption property of the polymer film 1 can be adjusted by changing the proportions of a first structural unit and a second structural unit contained in the polymer film 1. The adjustment method will be described later.

### First substance and second substance

The reaction between the first substance 5 and the second substance 4 may be a reaction that results in a change in the output from the sensor 2. Such a reaction may be, for example, a redox reaction, an enzymatic reaction, an antigen-antibody reaction, or a reaction in which the first substance 5 and the second substance 4 are bonded by chemical adsorption, intermolecular interaction, or ionic interaction, etc. Alternatively, the reaction between the first substance 5 and the second substance 4 may be a reaction that generates a new substance (third substance) by an enzymatic reaction or the like.

The second substance 4 fixed to the detection unit 23 may be selected as appropriate in accordance with the first substance 5. For example, when the first substance 5 is a specific protein, DNA, or cell in a sample, the second substance 4 may be an antibody, peptide, or aptamer. Also, for example, when the first substance 5 is an antibody, the second substance 4 may be an antigen. Also, for example, when the first substance 5 is a substrate, the second substance 4 may be an enzyme.

The measuring device 100 may indirectly detect the first substance 5, which is the target. For example, a substance similar to the first substance 5 may be fixed to the detection unit 23 as the second substance 4. That is, for example, antibodies whose antigens are the first substance 5 may be reacted with the first substance 5 in advance, and the unreacted antibodies may be reacted with the fixed second substance 4. In this case, the measuring device 100 can, for example, calculate the amount of the first substance 5 indirectly from the amount of antibodies detected, as long as the amount of the antibodies is known.

### Polymer

The polymer 3 constitutes the polymer film 1. Hereinafter, an example of the polymer 3 of the present disclosure (hereinafter, abbreviated as "polymer of the present embodiment") will be described.

The polymer according to the present embodiment is a copolymer of a compound represented by Formula (1) below and a compound represented by Formula (2) below. 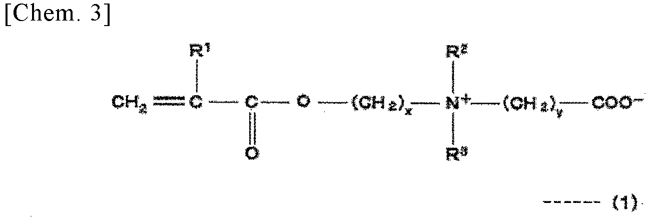 where in Formula (1),
R¹ is a hydrogen atom or a methyl group,
R² and R³ are each independently a hydrogen atom or an alkyl group having from 1 to 4 carbon atom(s), and
x and y are each independently an integer from 1 to 4 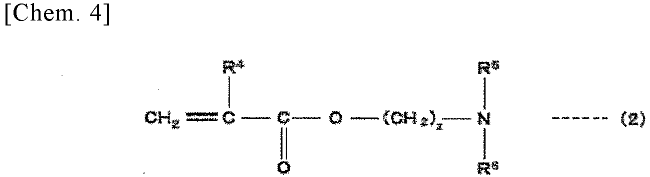 where in Formula (2),
R⁴ is a hydrogen atom or a methyl group,
R⁵ and R⁶ are each independently an alkyl group having from 1 to 4 carbon atom(s), and
z is an integer from 1 to 4.

Examples of the compound represented by Formula (1) include N-(carboxymethyl)-N,N-dimethyl-2-[(2-methyl-1-oxo-2-propen-1-yl)-oxy]etanaminium (CBMA1) and 2-carboxy-N,N-dimethyl-N-[2'-(methacryloyl)oxyethyl]etanaminium (CBMA2,3-[[2-(methacryloyloxy)ethyl]dimethylammonio]propionate). The compound represented by Formula (1) may be a commercially available product, or may be a compound obtained by synthesis.

Examples of the compound represented by Formula (2) include N,N-dimethylaminoethyl(meth)acrylate, N,N-diethylaminoethyl(meth)acrylate, and N,N-diisopropylaminoethyl(meth)acrylate. The compound represented by Formula (2) may be a commercially available product, or may be a compound obtained by synthesis.

A polymer film having a different non-specific adsorption property can be produced from the polymer of the present embodiment. Here, the zwitterionic structure in the structure illustrated in Formula (1) allows the polymer of the present embodiment to suppress non-specific adsorption. Furthermore, the dimethylamino group in the structure illustrated in Formula (2) allows the polymer of the present embodiment to promote non-specific adsorption. Thus, by adjusting the proportions of the two structures in the polymer of the embodiment, the non-specific adsorption property of the polymer as a polymer film can be adjusted.

When the polymer of the present embodiment is used in a measuring device such as a biosensor, the proportion of structural units derived from the compound represented by Formula (2) contained in the copolymer described above may be 5 mol% or less, or 2 mol% or less, from the perspective of suppressing non-specific adsorption.

When the polymer of the present embodiment is used in a non-detection region of a carrier for measurement such as an ELISA plate, the proportion of structural units derived from the compound represented by Formula (2) contained in the copolymer described above may be 20 mol% or greater, from the perspective of actively enhancing the non-specific adsorption property. The proportion of structural units derived from the compound represented by Formula (2) contained in the copolymer described above may also be 35 mol% or greater, or 50 mol% or greater.

Furthermore, the polymer of the present embodiment can also be expressed as including a first structural unit represented by Formula (3) below and a second structural unit (DMAEMA unit) represented by Formula (4) below. The first structural unit is a structural unit derived from the compound represented by Formula (1) above. The second structural unit is a structural unit derived from the compound represented by Formula (2) above. 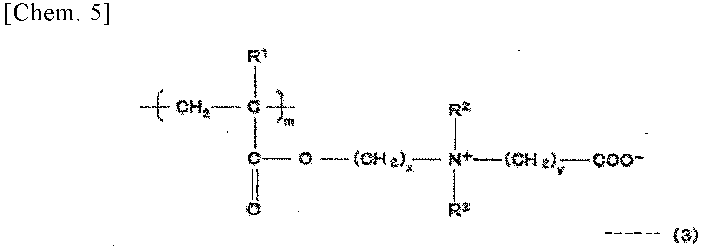 where in Formula (3),
R¹, R², R³, x, and y are the same as those of Formula (1) above, and
m is from 1 to 500 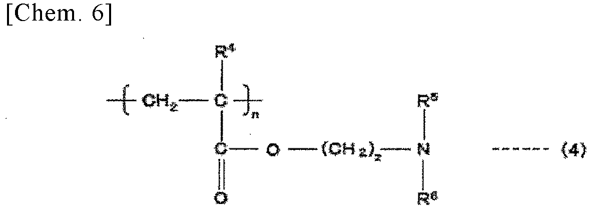 where in Formula (4),
R⁴, R⁵, R⁶, and z are the same as those of Formula (2) above, and
n is from 1 to 500.

The polymer of the present embodiment may have an active ester group at the end of a portion of the side chains of the first structural unit represented by Formula (3). Examples of the active ester group include a succinimide group.

At least one end of the main chain of the polymer of the present embodiment may have a thiol group or a dithioester group from the perspective of easily fixing the end to the polymer film fixed to the substrate 22 (measurement substrate 12).

When the polymer of the present embodiment is used in a measuring device such as a biosensor, m and n of Formulas (3) and (4) may satisfy the following relationship, from the perspective of suppressing non-specific adsorption. That is, n/(m + n) × 100 may be 5 or less, or may be 2 or less.

When the polymer of the present embodiment is used in a non-detection region of a carrier for measurement such as an ELISA plate, m and n of Formulas (3) and (4) may satisfy the following relationship, from the perspective of actively enhancing the non-specific adsorption property. That is, n/(m + n) × 100 may be 20 or greater, or 35 or greater.

A weight average molecular weight of the polymer of the present embodiment may be 3000 or greater, or 5000 or greater, from the perspective of film formation density. The weight average molecular weight of the polymer of the present embodiment may also be 100000 or less, or 70000 or less. Furthermore, the polymer of the present embodiment may be a random copolymer, a block copolymer, or a graft copolymer.

The weight average molecular weight of the polymer of the present embodiment may be 20000 or greater, from the perspective of suppressing the amount of non-specific adsorption in serum. Furthermore, the weight average molecular weight of the polymer of the present embodiment may be 30000 or less. In addition to the weight average molecular weight of the polymer of the present embodiment being within the range described above, when the proportion of structural units derived from the compound represented by Formula (2) contained in the polymer of the present embodiment is 7 mol% or less (or 5 mol% or less, or 3 mol% or less), the amount of non-specific adsorption in serum can be further suppressed.

The weight average molecular weight of the polymer of the present embodiment may be 2000 or greater, or 5000 or greater, from the perspective of suppressing the amount of non-specific adsorption in sweat or urine. Furthermore, the weight average molecular weight of the polymer of the present embodiment may be 20000 or less, or 10000 or less. In addition to the weight average molecular weight of the polymer of the present embodiment being within the range described above, when the proportion of structural units derived from the compound represented by Formula (2) contained in the polymer of the present embodiment is 7 mol% or less (or 5 mol% or less, or 3 mol% or less), the amount of non-specific adsorption in sweat or urine can be further suppressed.

A number average molecular weight or the weight average molecular weight of the polymer of the present embodiment can be determined by a known technique such as gel permeation chromatography (GPC).

The copolymer of a compound represented by Formula (1) above and a compound represented by Formula (2) above can be identified by a known organic analysis technique. For example, the copolymer may be identified by Nuclear Magnetic Resonance (NMR). Alternatively, for example, the copolymer may be identified using a gas chromatograph. Alternatively, for example, the copolymer may be identified using a liquid chromatograph. Alternatively, for example, the copolymer may be identified by laser Raman spectroscopy analysis. That is, when identifying the copolymer, devices capable of carrying out these techniques may be used. The identification technique and device are not limited to the techniques and devices described above as long as the copolymer can be identified.

The polymer of the present embodiment can be produced by a known polymerization method such as radical polymerization. For example, the compound represented by Formula (1) and the compound represented by Formula (2) are dissolved in a solvent. Then, polymerization reaction is performed by adding a polymerization catalyst and a polymerization initiator. The polymerization conditions such as the polymerization time, the polymerization temperature, and the polymerization solvent can be selected as appropriate depending on the type of the monomer, the amount to be used, the ratio to be used, and the like.

Examples of a method of fixing the polymer 3 to the substrate 22 include a method of applying a polymer solution, obtained by dissolving the polymer 3 in a solvent, on the substrate 22 and drying, graft polymerization by radiation or ultraviolet light, and chemical reaction with a functional group of the substrate 22. By these methods, a polymer film 1 including the polymer 3 is formed on the substrate 22.

Examples of a method of fixing the second substance 4 to the polymer 3 (polymer film 1) include a method of covalently bonding the second substance 4 to the carboxyl groups of the polymer 3. For example, the polymer 3 is reacted with N-hydroxysulfosuccinimide (NHS) and 1-ethyl-3-(3-dimethylaminopropyl)carbodiimide (EDC) (NHS/EDC activation). Then, the carboxyl groups contained in the polymer 3 are replaced by the NHS ester groups. By reacting the amino groups of the second substance 4 with the activated NHS ester groups, the second substance 4 is fixed to the polymer 3 (polymer film 1). The NHS/EDC activation of the polymer 3 may be performed before the polymer 3 is fixed to the substrate 22, or may be performed after the polymer 3 to is fixed the substrate 22.

### Embodiment 2

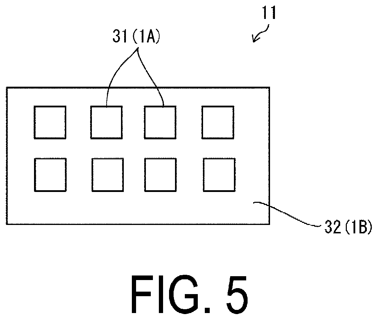

Next, a carrier for measurement according to an embodiment will be described using drawings when appropriate. For convenience of description, members having the same functions as those described in the embodiment above are denoted by the same reference signs, and descriptions thereof will not be repeated. FIG. 5 illustrates an overall configuration of a carrier for measurement 11 according to the present embodiment. Examples of the carrier for measurement 11 include a plate for ELISA (enzyme-linked immunosorbent assay).

The carrier for measurement 11 includes a detection region 31 and a non-detection region 32 on a surface of a measurement substrate 12, in which the detection region 31 specifically captures a target substance (first substance 5) contained in a sample while the non-detection region 32 non-selectively adsorbs a blocking agent and the like. A polymer film 1A is fixed to the detection region 31 while a polymer film 1B is fixed to the non-detection region 32.

Similar to the polymer film 1 described in Embodiment 1, a second substance 4 that reacts with the first substance 5 is fixed to the polymer film 1A. Furthermore, the polymer film 1A is adjusted to have a low non-specific adsorption property.

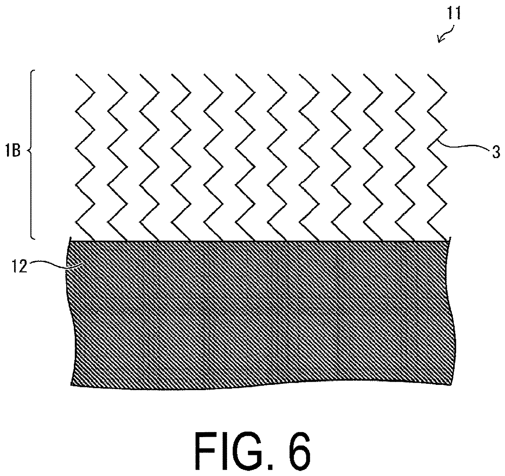

FIG. 6 conceptually illustrates a polymer film formed in the non-detection region 32. The second substance 4 is not fixed to the polymer film 1B, and only a polymer film 1 containing the polymer 3 is fixed to a surface of the measurement substrate 12. The polymer film 1B is a film adjusted to have a high non-specific adsorption property. That is, two types of polymer films 1A and 1B containing the same structural units (first and second structural units) but having different non-specific adsorption properties are formed on one carrier for measurement 11.

### Use example of carrier for measurement

First, a desired second substance 4 is fixed to the polymer film 1A in the detection region 31. Then, the non-detection region 32 is treated with a blocking agent, which is non-specifically adsorbed to the polymer film 1B in the non-detection region 32.

Thereafter, by bringing a sample into contact with the detection region 31, a first substance 5, which is the target substance contained in the sample, reacts with the second substance 4, and the reaction is detected by a detection reagent. Examples of the detection reagent include a redox substance, a fluorescent substance, an enzyme, and a dye compound. The polymer film 1B in the non-detection region 32 is treated with the blocking agent, resulting in effective suppression of non-specific adsorption.

The application of the polymer film 1B is not limited to the description above, and the polymer film 1B may be used to actively enhance the non-specific adsorption property.

### Measurement substrate

The measurement substrate 12 may be, for example: a metal such as gold, silver, copper, platinum, and aluminum; a plastic such as polyethylene and polypropylene; or an inorganic material such as titanium oxide, silica, glass, and ceramic. The measurement substrate 12 is not limited to these examples.

A shape of the measurement substrate 12 may be, for example, the shape of a plate, the shape of a particle, the shape of a micro structure, or the shape of a microtiter plate. The shape of the measurement substrate 12 is not limited to these examples.

### Measurement kit

A measurement kit including the measurement substrate 12 to which the polymer films 1A and 1B are fixed, the second substance 4, and the detection reagent is also included in the scope of the present disclosure. The second substance 4 may be fixed to the polymer film 1 in advance during the production of the product, or may be fixed to the polymer film 1 by the user prior to measurement.

The measurement kit according to the present embodiment may include another reagent or instrument. For example, a constituent in addition to the second substance 4 and the detection reagent may be provided. Further, a buffer or the like may be provided. Also, a plurality of different reagents in the measurement kit according to the present embodiment may be mixed in an appropriate volume and/or form, or may be provided in separate containers. Furthermore, the measurement kit according to the present embodiment may include instructions that describes, for example, a procedure for achieving detection of the reaction between the first substance 5 and the second substance 4. The instructions may be written or printed on paper or other media, or may be attached to readable discs, such as magnetic tapes and computers, or may be attached to electronic media such as CD-ROMs.

The invention according to the present disclosure has been described above based on drawings and examples. However, the invention according to the present disclosure is not limited to the embodiments described above. That is, the invention according to the present disclosure can be variously modified within the scope indicated by the present disclosure, and an embodiment obtained by appropriately combining the technical means disclosed in different embodiments is also within the technical scope of the invention according to the present disclosure. That is, it should be noted that those skilled in the art can easily make various variations or modifications based on the present disclosure. It should also be noted that these variations or modifications are within the scope of the present disclosure.

### Examples

In the following examples, "%" means "mass%" unless otherwise specified.

### Example 1: Preparation of polymer (copolymer)

A solvent (methanol and water) used for polymerization of polymer was deoxidized in advance. The monomers used were 3-[[2-(methacryloyloxy)ethyl]dimethylammoni]propionate (CBMA2, available from Tokyo Chemical Industry Co., Ltd.) and 2-(dimethylamino)ethyl methacrylate (DMAEMA, available from Tokyo Chemical Industry Co., Ltd.). The monomers were dissolved in the solvent (mixed solvent of methanol and water) to an arbitrary ratio of the monomers. After the dissolution, copper (I) bromide, serving as a catalyst, and 2,2'-bipyridyl, serving as a ligand, were added to the mixture and dissolved to give the following molar ratio: monomers:2,2'-bipyridyl:copper (I) bromide = 100:6:1. Finally, bis[2-(2'-bromoisobutyryloxy)ethyl] disulfide was added as a polymerization initiator in an amount of 0.5 mol% with respect to the amount of the monomers to carry out polymerization. Polymerization was performed at room temperature under a nitrogen atmosphere and stopped by opening the polymerization solution to the atmosphere; a portion of the polymerization solution was diluted and subjected to GPC measurement of molecular weight. The result of weight average molecular weight (Mw) measured was 38100. Thereafter, the obtained polymer solution was diluted with an appropriate solvent at an arbitrary multiplication factor and then applied onto an Au substrate. Then, the Au substrate was washed and dried, and a polymer film (polymer film 1) was formed.

The GPC measurement was carried out under the following measurement conditions. A sample was diluted with a 10 mM LiBr aqueous solution to approximately 0.1 mass% and dispersed in an ultrasonic disperser.
Device: High Performance Liquid Chromatograph LC-20, available from Shimadzu Corporation
Column performance: Mw 20000000 to Mw 100
Column: TSKgel G4000PWX_{L}
Measuring solvent: LiBr 10 mM aqueous solution
Detector: RID-10A
Column temperature: 40°C
Flow rate: 1.0 mL/min
Injection volume: 100 µL

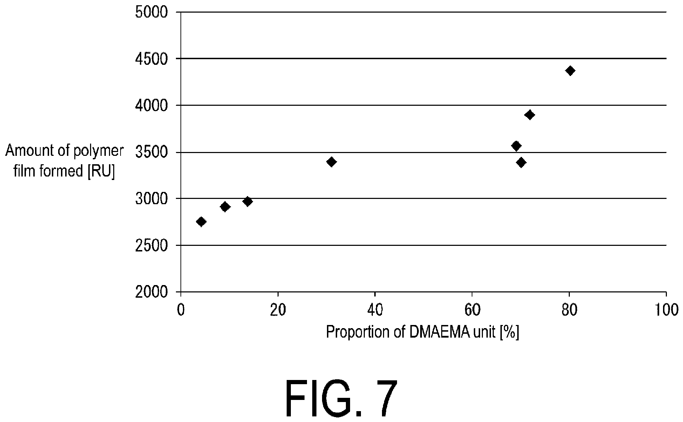

FIG. 7 illustrates a graph plotted by taking the proportion (%) of the DMAEMA units (second structural units) in the polymer prepared in Example 1 on the horizontal axis and the amount of polymer film formed (RU) on the Au substrate on the vertical axis. The proportion (%) of the DMAEMA units was calculated based on Formula (A) below. The amount of polymer film formed (RU) was measured by a SPR apparatus T-200 (available from GE Healthcare). Amount of DMAEMA added/(Amount of CBMA2 added + Amount of DMAEMA added) ... (A)

The polymer film was formed using a sensor chip of a SIA kit Au (available from GE Healthcare). The sensor chip was then washed with, for example, a piranha solution, and placed in the SPR apparatus. Next, an arbitrary polymer solution was injected at an appropriate flow rate over a desired time. The difference in the signal before and after the injection was taken as the amount of polymer film formed.

### Evaluation Example 1: Measurement of amount of non-specific adsorption of serum

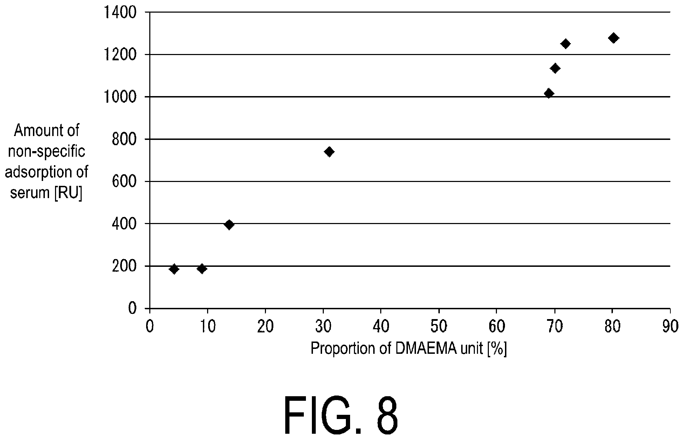

Human serum was brought into contact with the polymer film on the Au substrate prepared in Example 1 to measure the amount of non-specific adsorption of serum constituents. The amount of non-specific adsorption of serum (RU) was measured by a SPR apparatus T-200 (available from GE Healthcare). The sensor chip with the polymer film formed was placed in the SPR apparatus; then, human serum was injected at an appropriate flow rate over a desired time. Thereafter, only a running buffer was run at the same flow rate over the same time as with the human serum. The difference in the signal before injecting the human serum and after running the running buffer was taken as the amount of non-specific adsorption of serum. The measurement results are presented in FIG. 8.

As illustrated in FIG. 8, the lower the proportion of the DMAEMA units (second structural units) in the polymer, the more the amount of non-specific adsorption of serum was suppressed. Therefore, it was found that a polymer having a different non-specific adsorption property can be synthesized based on the proportions of CBMA2 and DMAEMA during the copolymerization of CBMA2 and DMAEMA, and that by using the polymer, the non-specific adsorption property of a polymer film can be adjusted.

### Evaluation Example 2: Relationship between hydrolysis time and DMAEMA unit ratio

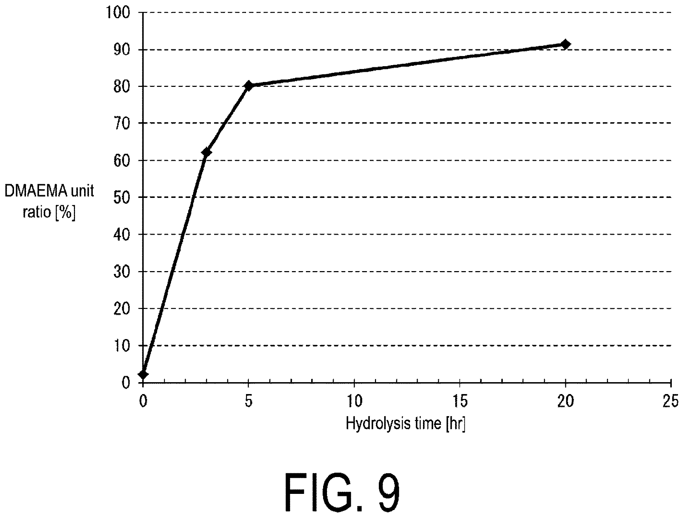

Powders of CBMA2 polymer were dissolved in a 100 mM NaOH to a concentration of 20 mg/mL, and hydrolysis was performed. The hydrolysis was performed at room temperature. The hydrolysis times were 0, 3, 5, and 20 hours. After the completion of hydrolysis, the amounts of DMAEMA in the polymers were measured. The measurement results are presented in FIG. 9.

As illustrated in FIG. 9, it was found that the amount of DMAEMA units in the polymer may be adjusted based on the hydrolysis time of the CBMA2 polymer. That is, it was found that by adjusting the hydrolysis time of the CBMA2 polymer, the proportions of the first structural units and the second structural units in the polymer 3 constituting the polymer film 1 can be adjusted, and the non-specific adsorption property of the polymer film 1 can be adjusted.

### Evaluation Example 3: Evaluation by NMR spectroscopy

The polymers prepared in Example 1 and Evaluation Example 2 were measured using NMR spectroscopy under the following measurement conditions, and the ratios of CBMA2 to DMAEMA were calculated.
Measuring device: ADVANCE III HD 400, available from Bruker BioSpin K.K.
Measurement conditions: Solvent: Heavy water
Temperature: 30°C
Criteria: H₂O peak is 4.70 ppm
Accumulation: 300 times
Method of calculating ratio of CBMA2 to DMAEMA:
   The ratios of CBMA2 to DMAEMA were calculated based on the ratios of a peak area near 3.2 ppm derived from protons bonded to the methyl group of the quaternary amine in the CBMA2 units to a peak area near 2.9 ppm derived from protons bonded to the methyl group of the tertiary amine in the DMAEMA units.

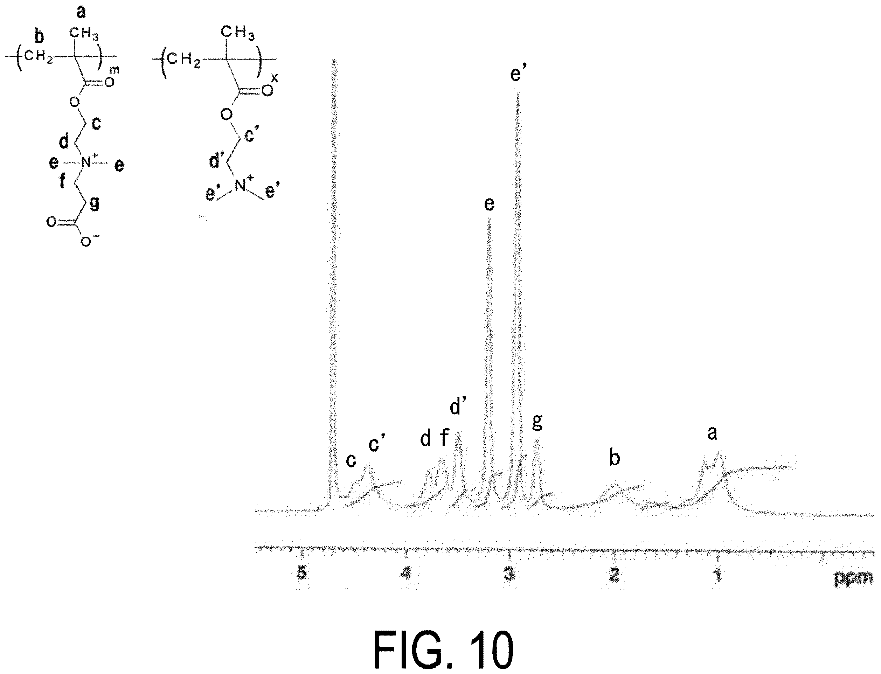

An example of the evaluation results by NMR spectroscopy is presented in FIG. 10. In FIG. 10, the ratio of CBMA2 to DMAEMA of the polymer measured was 4 to 6 (CBMA2/DMAEMA = 4/6).

### Evaluation Example 4: Examination of polymerization conditions for polymer

The polymerization conditions for obtaining a polymer having a desired molecular weight were examined. A solvent (methanol and water) used for polymerization of polymer was deoxidized in advance. CBMA2 was used as a monomer. The monomer was dissolved in the solvent (mixed solvent of methanol and water). After the dissolution, copper (I) bromide (CuBr) and 2,2'-bipyridyl (bpy) were added and dissolved to give the molar ratios [monomer:copper (I) bromide:2,2'-bipyridyl] presented in Table 1. Finally, as a polymerization initiator, bis[2-(2'-bromoisobutyryloxy)ethyl]disulfide (BiBOEDS) was added to give the molar ratios presented in Table 1, and polymerization was performed for 6 hours. The polymerization was performed at room temperature under a nitrogen atmosphere and stopped by opening the polymerization solutions to the atmosphere; a portion of the polymerization solutions were diluted and subjected to GPC measurement for molecular weight under the same conditions as in Example 1.

**[Table 1]**

| CBMA2 | bpy | CuBr | BiBEDS | Polymer molecular weight | |
|---|---|---|---|---|---|
| | | | | Mn | Mw |
| 100 | 3 | 0.5 | 0.25 | 40,200 | 66,900 |
| 100 | 6 | 1 | 0.5 | 22,800 | 38,100 |
| 100 | 12 | 2 | 1 | 15,500 | 25,100 |
| 100 | 30 | 5 | 2.5 | 8,200 | 13,900 |
| 100 | 60 | 10 | 5 | 5,300 | 8,400 |

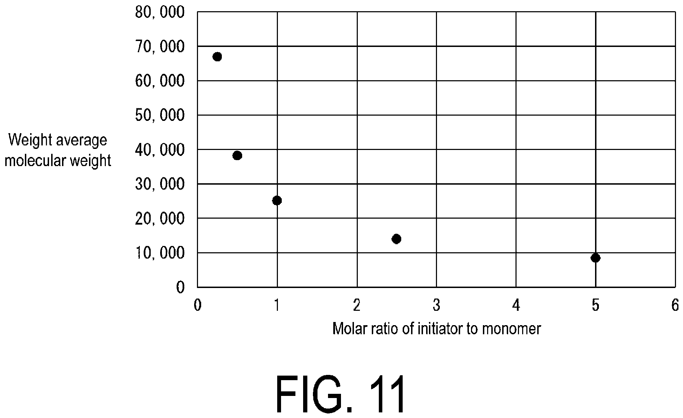

The examination results are presented in Table 1 and FIG. 11. The vertical axis in FIG. 11 indicates the weight average molecular weight of polymer, while the horizontal axis indicates the molar ratio of BiBOEDS to CBMA2. The number average molecular weight (Mn) and the weight average molecular weight (Mw) were measured by gel permeation chromatography (GPC) under the same conditions as in Example 1.

### Evaluation Example 5: Evaluation of amount of non-specific adsorption of serum or urine due to changes in molecular weight of polymer

Based on the examination results of polymerization conditions for polymer in Evaluation Example 4, polymerization was performed to give polymers having a weight average molecular weight of approximately 10000, approximately 20000, approximately 35000, and approximately 60000. Polymerization was performed in the same procedure as in Example 1 except that the amounts of monomer (CBMA2 and DMAEMA), CuBr, bby and BiBOEDS charged were prepared. In addition, polymerization was performed to give a proportion of the DMAEMA units (second structural units) in polymer to be from 2 to 3%. The weight average molecular weights of the polymerized polymers were measured by GPC. Then, polymer films (polymer films 1) were formed on Au substrates in the same procedure as in Example 1. The proportions of the DMAEMA units (second structural units) in polymers were calculated by the method described in Evaluation Example 3.

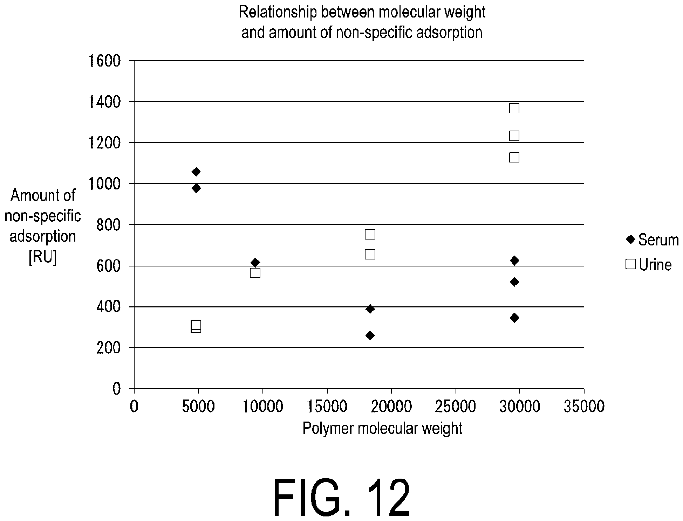

Measurement targets (human serum or human urine) were brought into contact with the polymer films on the Au substrates produced, and the amounts of non-specific adsorption of the target measurement constituent were measured. The amounts of non-specific adsorption (RU) were measured by a SPR apparatus T-200 (available from GE Healthcare). Sensor chips with polymer films formed were placed in the SPR apparatus, and the measurement targets were then injected at an appropriate flow rate over a desired time. Thereafter, only running buffers were run at the same flow rate over the same time as with the measurement targets. The differences in the signals before injecting the measurement targets and after running the running buffers were taken as the amounts of non-specific adsorption. The measurement results are presented in FIG. 12. The horizontal axis in FIG. 12 is the weight average molecular weight (Mw) of polymers in polymer film, predicted based on the weight average molecular weight of polymerized polymer. The polymerized polymers use BiBOEDS having a disulfide bond as the polymerization initiator; as such, when a polymer film is formed on an Au substrate, the polymer is decomposed into two by the reaction between gold and a BiBOEDS-derived disulfide bond. Since the molecular weights of the two polymers after the decomposition (polymers in polymer film) were substantially the same, the value obtained by dividing the weight average molecular weight of the polymerized polymer by 2 was taken as the weight average molecular weight (Mw) of the polymers in polymer film.

As illustrated in FIG. 12, when the measurement target was urine, the amount of non-specific adsorption of urine was suppressed when the weight average molecular weight of the polymer was approximately 20000 or less; the lower the weight average molecular weight, the more the amount of non-specific adsorption of urine was suppressed. Meanwhile, when the measurement target was serum, the amount of non-specific adsorption of serum was suppressed when the weight average molecular weight of the polymer was 20000 or greater. From Evaluation Example 5, it was found that the range of weight average molecular weight of the polymer that can suppress the amount of non-specific adsorption differs depending on the measurement target.

From the above examples and evaluation examples, it has been demonstrated that a polymer film having a different non-specific adsorption property can be produced using the copolymer of a compound represented by Formula (1) above and a compound represented by Formula (2).

### Industrial Applicability

The present disclosure can be used in a measuring device or a measuring plate provided with a detection unit in which a polymer film is formed.

### Reference Signs List

1, 1A, 1B Polymer film
2 Sensor
3 Polymer
4 Second substance
5 First substance
6 Control device
11 Carrier for measurement
12 Measurement substrate
21 External terminal
22 Substrate
23 Detection unit
24 Reference unit
25a First IDT electrode
25b Second IDT electrode
26 Flow path member
27 Supply port
28 Discharge port
31 Detection region
32 Non-detection region
100 Measuring device

## Claims

1. A copolymer of a compound represented by Formula (1) and a compound represented by Formula (2): 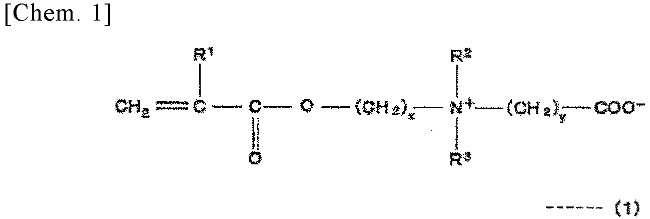
where in Formula (1),
R¹ is a hydrogen atom or a methyl group,
R² and R³ are each independently a hydrogen atom or an alkyl group having from 1 to 4 carbon atom(s), and
x and y are each independently an integer from 1 to 4, 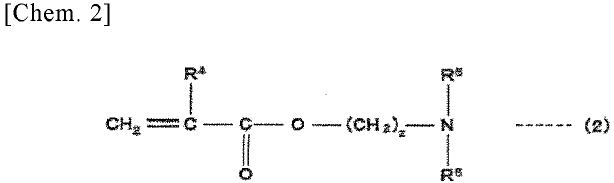 where in Formula (2),
R⁴ is a hydrogen atom or a methyl group,
R⁵ and R⁶ are each independently an alkyl group having from 1 to 4 carbon atom(s), and
z is an integer from 1 to 4.

2. The copolymer according to claim 1, containing a proportion of a structural unit derived from the compound represented by Formula (2) of 5 mol% or less.

3. The copolymer according to claim 1, containing a proportion of a structural unit derived from the compound represented by Formula (2) of 20 mol% or greater.

4. The copolymer according to claim 2, having a weight average molecular weight of 2000 or greater and 20000 or less.

5. The copolymer according to claim 2, having a weight average molecular weight of 20000 or greater.

6. The copolymer according to any one of claims 2 to 5, wherein at least one end of the copolymer contains a thiol group or a dithioester group.

7. A measuring device comprising a polymer film containing the copolymer according to any one of claims 1 to 6.

8. A carrier for measurement comprising a polymer film containing the copolymer according to any one of claims 1 to 6.
